# EUROPÄISCHE PATENTANMELDUNG

(11) **EP 2 484 753 A1**
(43) Veröffentlichungstag der Anmeldung: **08.08.2012**
(21) Anmeldenummer: 12000532.7
(22) Anmeldetag: 27.01.2012
(51) Int. Cl.: C12N 1/12, C12M 1/00

(54) **Verfahren und Vorrichtung zur Biomasseerzeugung**

(30) Priorität: 04.02.2011 EP 11000888
(71) Anmelder: Universität Duisburg-Essen, 45141 Essen (DE); SEE-O-TWO Patentgesellschaft mbH, 1130 Wien (AT)
(72) Erfinder: Franke, Hilmar, 49565 Duisburg (DE)
(74) Vertreter: Häckel, Stefan

(57) **Zusammenfassung**

Die vorliegende Erfindung betrifft ein Verfahren zur Biomasseerzeugung, wobei es sich bei der Biomasse vorzugsweise um eine lebende Spezies, insbesondere Algen, handelt, und wobei die Biomasse mittels gravimetrischer Separation vom Medium getrennt wird. Weiterhin betrifft die vorliegende Erfindung eine Vorrichtung zur Biomasseerzeugung, wobei es sich bei der Biomasse vorzugsweise im eine lebende Spezies, insbesondere Algen, handelt, und wobei die Vorrichtung die gravimetrische Separation der Biomasse vom Medium ermöglicht.

## Beschreibung

Die vorliegende Erfindung betrifft ein Verfahren zur Biomasseerzeugung gemäß dem Oberbegriff des Patentanspruchs 1. Weiterhin betrifft die vorliegende Erfindung eine Vorrichtung zur Biomasseerzeugung gemäß dem Oberbegriff des Patentanspruchs 8.

Die vorliegende Erfindung befasst sich insbesondere mit der Nachahmung der Photosynthese zur Bindung von Kohlendioxid bzw. dessen Umwandlung in Biomasse. Es ist bekannt, eine Algensuspension zu beleuchten, um das Wachstum der Algen anzuregen. Die Algen verbrauchen Kohlendioxid zum Wachstum und zur Bildung von Biomasse. Eine derartige Anordnung zur Biomasseerzeugung ist beispielsweise aus der WO 2008/135276 A2 bekannt.

Problematisch ist die Abtrennung der Algen von der Algensuspension bzw. dem wässrigen Medium, da die Algen nur in sehr geringer Konzentration im Medium vorliegen. Die WO 2010/136195 A1 beschreibt eine elektrische oder elektrochemische Separation der Algen vom Medium bzw, Wasser, Bei diesem Verfahren erfolgt eine teilweise Entfernung des Mediums. Zur vollständigen Trocknung der Algen können weitere Verfahrensschritte folgen.

Die bislang bekannten Verfahren zur Separation von Algen sind einerseits aus energieökonomischer als auch verfahrenstechnischer Sichtweise sehr aufwändig. Die WO 2010/104867 A1 beschreibt das Trocknen der Algen durch Aufpressen auf ein Filtermaterial. Alternativ besteht die Möglichkeit in der Gefriertrocknung, Jedoch sind diese Verfahren zur Entfernung großer Wassermengen insbesondere im Hinblick auf die Energieeffizienz unwirtschaftlich.

Der vorliegenden Erfindung liegt die Aufgabe zugrunde, ein Verfahren bzw. eine Vorrichtung zur Biomasseerzeugung anzugeben, wobei die Biomasse bzw. eine Spezies, insbesondere Algen, effizienter und energiesparender abgetrennt werden kann bzw. können.

Die obige Aufgabe wird durch ein Verfahren gemäß Anspruch 1 oder eine Vorrichtung gemäß Anspruch 8 gelöst. Vorteilhafte Weiterbildungen sind Gegenstand der Unteransprüche,

Ein Aspekt der vorliegenden Erfindung besteht darin, die vorzugsweise lebende Spezies, wie Algen, nach thermischer Induktion des Zelltodes unter Nutzung der Gravitationskraft vom wässrigen Medium, also gravimetrisch, zu separieren. Dieses Verfahren ist äußerst unkompliziert in seiner Handhabung, da die Spezies unmittelbar nach dem Absterben aufgrund des Verlusts der Motilität sedimentiert und somit leicht vom Medium abgetrennt werden kann. Auf diesem Weg ist es möglich, bis zu 95 Gew.-% des vorzugsweise wässrigen oder primär aus Wasser bestehenden Mediums von der Spezies abzutrennen.

Unter "Zelltod" ist vorzugsweise auch eine - ggf. sogar nur zeitweise - Beeinträchtigung oder Verringerung der Zellvitalität oder -aktivität zu verstehen, die zu einem Absinken der Zellen bzw. Spezies bei der Sedimentation führt.

Vorzugsweise wird der Zelltod der Spezies durch Erwärmung induziert. Durch die Erwärmung werden die Vitalfunktionen der Zellen infolge der Denaturierung lebensnotwendiger Enzyme und Proteine zumindest zeitweise derart beeinträchtigt, dass die Spezies, insbesondere die Algen, nicht mehr an der Oberfläche des wässrigen Mediums schwimmen kann und sedimentiert.

Besonders bevorzugt wird die Spezies solarthermisch, beispielsweise mittels einer Parabolrinne, erwärmt, was insbesondere hinsichtlich der Energiebilanz vorteilhaft ist.

Die Sedimentation der Spezies kann praktisch in einem beliebigen erfolgen, wobei es besonders vorteilhaft ist, wenn der Behälter im unteren Bereich eine Auslassöffnung zur Entnahme der (aufkonzentrierten) Biomasse aufweist. Auch hierfür muss zumindest im Wesentlichen keine externe Energie, wie für ein Zentrifugieren, zugeführt werden, da sich das Verfahren die Gravitationskraft zunutze macht.

Neben der besonders unkomplizierten Verfahrensführung und einem einfachen Aufbau ist es somit insbesondere vorteilhaft, dass die Separation bei einer guten Energiebilanz betrieben werden kann, da die thermische Induktion des Zelltodes besonders bevorzugt durch Erwärmung unter Nutzung solarthermischer Energie erfolgt, und der Sedimentationsprozess zumindest im Wesentlichen ebenfalls keine externe Energiezufuhr benötigt.

Weitere Aspekte, Vorteile, Merkmale und Eigenschaften der vorliegenden Erfindung ergeben sich aus den Ansprüchen und der nachfolgenden Beschreibung einer bevorzugten Ausführungsform anhand der Zeichnung. Es zeigt:
- Fig. 1: eine schematische Darstellung einer vorschlagsgemäßen Einrichtung zur Kultivierung einer Spezies, insbesondere Algen;
- Fig. 2: eine schematische Darstellung einer vorschlagsgemäßen Vorrichtung zur Biomasseerzeugung; und
- Fig. 3: eine schematische detailliertere Darstellung der Vorrichtung.

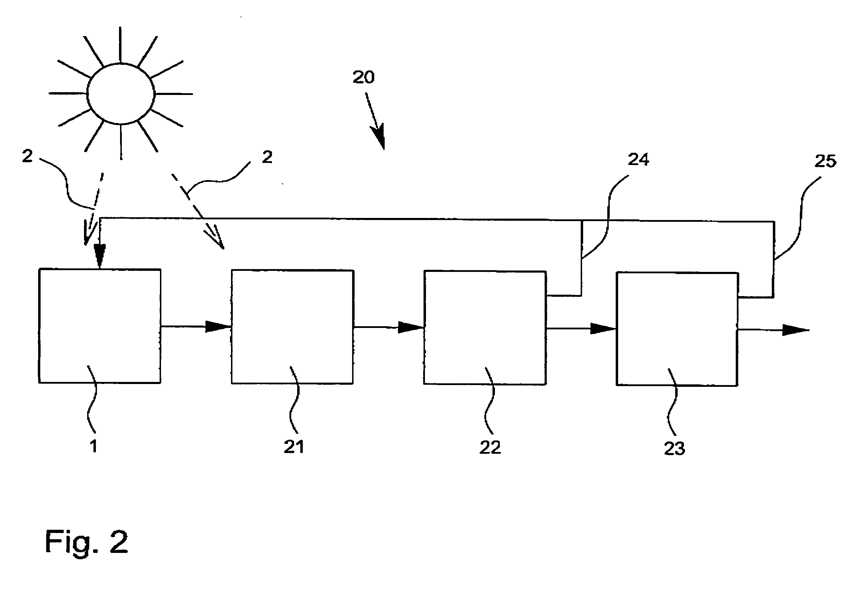

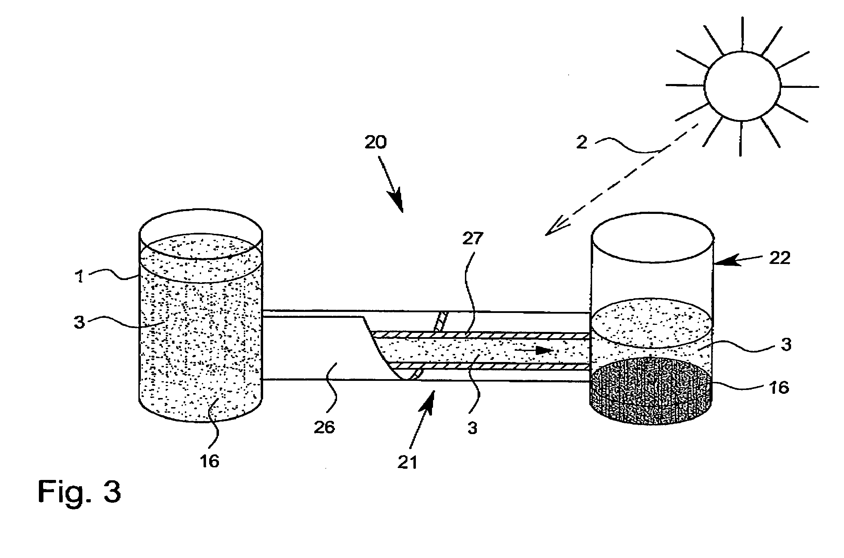

Nachfolgend wird zunächst eine bevorzugte Ausführungsform einer Einrichtung 1 zur Biomasseerzeugung, insbesondere zur Kultivierung bzw. Vermehrung einer lebenden Spezies 16, wie Algen, in einem Medium 3 erläutert. Die erfindungsgemäße Separation der Spezies 16 vom Medium 3 sowie der bevorzugte Aufbau einer entsprechenden Vorrichtung 20 zur Separation werden dann anhand von Fig. 2 und 3 erläutert.

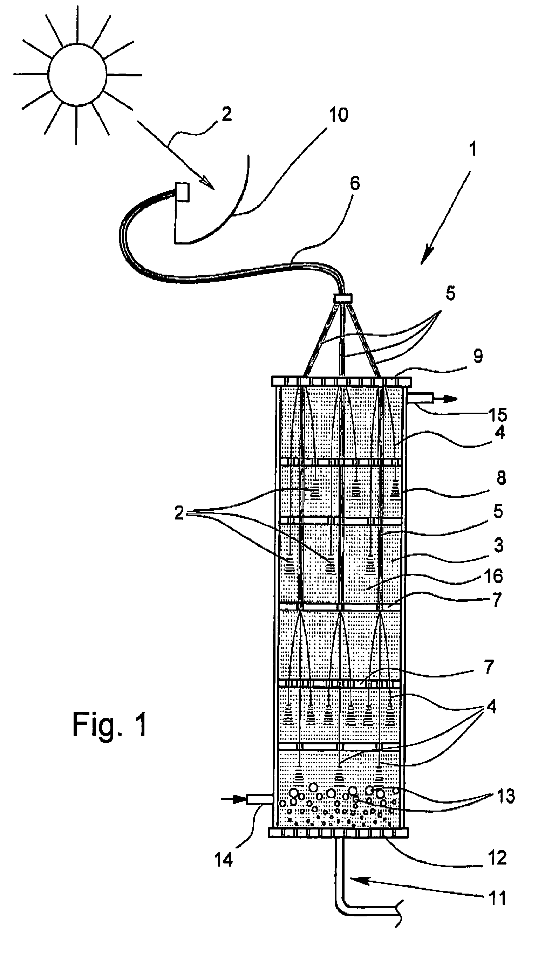

Fig. 1 zeigt in einer schematischen, nicht maßstabsgerechten Darstellung die Einrichtung 1 zur Biomasseerzeugung. Die Einrichtung 1 dient der Biomasseerzeugung, wobei Licht 2 und/oder Kohlendioxid bzw. Kohlenwasserstoffe einem flüssigen Medium 3 zugeführt werden, das mindestens eine lebende Spezies 16 enthält.

Dem Medium 3 wird besonders bevorzugt Kohlendioxid, insbesondere als Gas, ggf. auch als Komponente eines Gasgemisches, und/oder auf sonstige Weise, beispielsweise in flüssiger bzw. gelöster Form zugeführt. Alternativ oder zusätzlich können auch sonstige Kohlenwasserstoffe dem Medium 3 zugeführt werden. Dies kann in entsprechender Weise oder anstatt der Zuführung von Kohlendioxid erfolgen. Dementsprechend gelten die Ausführungen zu der Zuführung von Kohlendioxid auch in entsprechender Weise für die Zuführung von sonstigen Kohlenwasserstoffen. In der nachfolgenden Beschreibung wird der Einfachheit halber jedoch oft nur von der Zuführung von Kohlendioxid gesprochen.

Bei dem Medium 3 handelt es sich insbesondere um eine Suspension oder dergleichen. Dementsprechend ist der Begriff "flüssig" in einem weiten Sinne zu verstehen, so dass insbesondere Suspensionen, Dispersionen oder sonstige Gemische oder Stoffe mit flüssigen Phasen oder Anteilen umfasst sind.

Das Medium 3 ist vorzugsweise photoaktiv und/oder biologisch aktiv. Insbesondere kann in dem Medium 3 eine Photosynthese oder eine sonstige Licht 2 und/oder Kohlendioxid benötigende Reaktion ablaufen. Insbesondere enthält das Medium 3 hierzu eine biologisch aktive Spezies 16, insbesondere Algen, sonstige Bakterien oder dergleichen.

Insbesondere ist das Medium 3 wässrig bzw. enthält Wasser und/oder ist stark lichtbestreuend. Besonders bevorzugt wird eine Algensuspension als Medium 3 eingesetzt. Insbesondere enthält das Medium 3 als Spezies 16 eine Algenmischkultur, die vorzugsweise zumindest in ähnlicher Form in Flüssen, Teichen oder dergleichen vorkommt. Derartige Mischkulturen sind nämlich besonders resistent gegen Umwelteinflüsse, Krankheiten und/oder sonstige Störungen.

Da die Einrichtung 1 vorzugsweise mit Algen bzw. mit einer Algensuspension als Spezies 16 bzw. Medium 3 eingesetzt wird, wird der folgenden Beschreibung primär auf das durch das eingeleitete Licht 2 induzierte Algenwachstum abgestellt. Jedoch gelten diese Ausführungen entsprechend auch für sonstige Spezies oder photo- bzw. bioaktive Medien 3.

Die Einrichtung 1 weist vorzugsweise eine Beleuchtungseinrichtung, hier mit lichtleitenden Fasern 4, zur Zuleitung des Lichts 2 auf. Bei der ersten Ausführungsform enden mehrere oder alle Fasern 4 in verschiedenen Raumbereichen, insbesondere zumindest teilweise in unterschiedlichen Tiefen und/oder Vertikalebenen, in dem Medium 3. Dies gestattet eine dreidimensionale Verteilung des Lichts 2 in dem Medium 3, wie in Fig. 1 beispielhaft schematisch angedeutet.

Vorzugsweise sind die Fasern 4 flexibel. Jedoch können grundsätzlich auch starre lichtleitende Stäbe - zumindest abschnittsweise und/oder am Austrittsende ins Medium 3 - eingesetzt werden.
Besonders bevorzugt enden die Fasern 4 in verschiedenen Ebenen innerhalb des Mediums 3, Jedoch können die Fasern 4 auch alle unterschiedlich oder unregelmäßig enden.

Ausgehend von einem Bündel (Hauptbündel) 5 von Fasern 4 erfolgt insbesondere eine Verzweigung oder Verästelung in Einzelbündel 6 und/oder Einzelfasem 4. Um eine optimale Verteilung des Lichts 2 in dem Medium 3 zu erreichen, enden die Fasern 4 vorzugsweise einzeln, also separat voneinander in dem Medium 3. Jedoch können auch mehrere Fasern 4 zusammen, beispielsweise als Einzelbündel 5, in dem Medium 3 enden.

Das Licht 2 tritt zumindest im wesentlichen oder ausschließlich am Ende der jeweiligen Faser 4 aus und beleuchtet das Medium 3 im jeweiligen Raumbereich, wie in Fig. 1 angedeutet.

Vorzugsweise weist die Einrichtung 1 insbesondere gitter- oder stangenartige Halterungen 7 in dem Medium 3 auf, die insbesondere in verschiedenen Ebenen oder Tiefen angeordnet sind, um die Fasern 4 bzw. Bündel 5, 6 zu halten oder zu führen.

Besonders bevorzugt bilden die Halterungen 7 Zwischenböden in einem Behälter oder Tank 8 mit dem Medium 3, um die Fasern 4 oder Bündel 5 oder 6 in verschiedenen Raumbereichen innerhalb des Mediums 3 enden zu lassen.

Beim Darstellungsbeispiel wird vorzugsweise Sonnenlicht zur Beleuchtung des Mediums 3 bzw. der Algen eingesetzt, auch wenn grundsätzlich jedes sonstige Licht verwendet werden kann. In diesem Zusammenhang ist anzumerken, dass der Begriff "Licht" vorzugsweise in einem weiten Sinne dahingehend zu verstehen ist, dass nicht nur sichtbares Licht, sondern beispielsweise alternativ oder zusätzlich auch Infrarot-Licht und/oder ultraviolettes Licht entsprechend dem Medium 3 zugeführt werden kann. Generell kann die Energiezuführung über die Fasern 4 des Medium 3 auch anderen Zwecken als der beim Darstellungsbeispiel vorgesehenen Photosynthese und/oder Anregung des biologischen Wachstums und/oder der Umwandlung von Kohlendioxid in Biomasse dienen.

Die Einrichtung 1 bzw. Beleuchtungseinrichtung weist vorzugsweise eine Lichtsammeleinrichtung, insbesondere einen Lichttrichter oder Kollektorspiegel 10, zum Auffangen von Sonnenlicht 2 auf, wie in Fig. 1 schematisch dargestellt. Das aufgefangene Sonnenlicht 2 oder sonstiges Licht kann dann über die Fasern 4 bzw. insbesondere ein Bündel 5 oder 6 zu dem Behälter bzw. Tank 8 mit dem Medium 3 bzw. in das Medium 3 mit insbesondere sehr geringen Verlusten geleitet werden,

Die Einrichtung 1 weist vorzugsweise eine Versorgungseinrichtung 11 zur Zuführung von Kohlendioxid und/oder Kohlenwasserstoffen, insbesondere zur Einleitung von Gas, das insbesondere Kohlendioxid enthält oder daraus besteht, auf. Beispielsweise kann Rauchgas, Klärgas, Faulgas, Luft oder dergleichen eingeleitet werden. Beim Darstellungsbeispiel weist die Einrichtung 11 insbesondere einen Boden, ein Sieb 12 oder ein sonstiges geeignetes Einleitungsmittel auf, um das Gas insbesondere in Form von Gasblasen 13 in das Medium 3 einzuleiten bzw. abzugeben, wie in Fig. 1 schematisch angedeutet.

Alternativ oder zusätzlich kann die Zuführung von Kohlendioxid bzw. Kohlenwasserstoffen auch in flüssiger bzw. wässriger oder gelöster Form erfolgen. Diese Art der Zuführung kann auch mit der gasförmigen Zuführung gekoppelt werden.

Das Medium 3 mit der Spezies 16 - also die Algensuspension - wird vorzugsweise durch einen Bereich oder eine ggf. auch separate Einrichtung zur zumindest teilweisen Abtrennung bzw. Entnahme der Spezies 16 zirkuliert. Hierzu weist der Tank 8 beispielsweise einen Zulauf 14 und einen Ablauf 15 auf, die in Fig. 1 schematisch angedeutet sind.

Die angesprochene Zirkulation und/oder eine Umwälzung dient vorzugsweise einer Anregung des Algenwachstums.

Bei der Einrichtung 1 zur Biomasseerzeugung handelt es sich vorzugsweise um einen Photobioreaktor, der das Medium 3 mit der Biomasse bzw. Spezies 16, insbesondere den Algen, enthält bzw. zu deren Kultivierung genutzt wird. Vorzugsweise wird die Erzeugung der Biomasse durch Licht 2 induziert.

Die Erzeugung der Biomasse kann in einer Batch-Kultur (statische Kultur) und/oder in einer Dauerkultur (kontinuierliche Kultur) erfolgen. Im Falle einer Batch-Kultur wird die Spezies 16 in das Medium 3 eingebracht, wobei das Medium 3 während der gesamten Anzucht von außen nicht verändert wird, das heißt es wird weder aufgefüllt, noch ergänzt oder ausgewechselt. Mit Erreichen der stationären Wachstumsphase wird die gesamte Kultur der Spezies 16 geerntet. Bei einer Dauerkultur hingegen wird die Spezies 16 kontinuierlich bereitgestellt, da regelmäßig CO₂ sowie weitere zum Wachstum erforderliche Nährstoffe in das Medium 3 geleitet werden. Auch kann das Medium 3 bei einer kontinuierlichen Kultur vollständig ausgetauscht werden, um ggf. toxische Stoffwechselprodukte der Spezies 16 zu entfernen.

Weiter ist es aber auch erforderlich, die erzeugte Biomasse bzw. Spezies 16, wie die Algen, in dem Medium 3 aufzukonzentrieren und/oder davon zu separieren, um die Biomasse abzutrennen und für weitere Anwendungen verfügbar zu machen und/oder effektiv trocknen zu können.

Nachfolgend wird anhand von Fig. 2 eine erfindungsgemäße Vorrichtung 20 zur Biomasseerzeugung erläutert, die neben einer Einrichtung 21 zur thermischen Induktion des Zelltodes vorzugsweise auch die Einrichtung 1 zur Biomasseerzeugung insbesondere in der oben beschriebenen oder in einer andersartigen Ausführungsform, eine Sedimentationseinrichtung 22 und/oder eine Trocknungseinrichtung 23 aufweist.

Die Einrichtung 21 zur thermischen Induktion des Zelltodes ist vorzugsweise direkt an die Einrichtung 1 zur Biomasseerzeugung angeschlossen. Es kann sich aber auch um eine hiervon separate Einrichtung handeln.

Die thermische Induktion des Zelltodes kann erfindungsgemäß durch eine Temperaturerhöhung und/oder eine Temperaturerniedrigung erfolgen. Vorzugsweise erfolgt die thermische Induktion des Zelltodes durch Erwärmung, also eine Temperaturerhöhung, in der Einrichtung 21. Insbesondere erfolgt eine Erwärmung der Spezies 16 auf mindestens 40 °C, bevorzugt auf mindestens 50 °C. Temperaturen von mehr als 40 °C fuhren bereits bei den meisten Spezies 16 bzw. Algen innerhalb kurzer Zeit zum Tod der Spezies, da die zum Erhalt der Vitalfunktionen erforderlichen Enzyme und Proteine denaturieren und somit nicht mehr ihre Funktion ausüben können.

Die Sterberate der Spezies 16 nimmt - ausgehend von 40 °C - mit steigender Temperatur zu. Vorzugsweise beträgt die Geschwindigkeit der Erwärmung mindestens 1 °C/s, insbesondere mindestens 10 °C/s.

Die Spezies 16 wird mittels der Einrichtung 21 vorzugsweise für mindestens 10 min, insbesondere jedoch mindestens 30 min oder mindestens 60 min, erwärmt.

Besonders bevorzugt erfolgt die Erwärmung mittels Sonneneinstrahlung bzw. Licht 2 oder Mikrowellenstrahlung, wie in Fig. 2 angedeutet.

Auch ist es möglich, die Spezies durch eine Temperaturerniedrigung auf unter 10 °C, vorzugsweise auf unter 1 °C, abzutöten bzw. die Vitalfunktionen soweit zu inaktivieren, dass die Spezies 16 in Folge des Motilitätsverlusts sedimentiert.

Alternativ oder zusätzlich zur thermischen Induktion sind prinzipiell auch andere Methoden einsetzbar, welche ebenfalls den Zelltod induzieren, wie UV-Einstrahlung oder Ultraschalleinwirkung.

Die Induktion des Zelltodes kann kontinuierlich oder diskontinuierlich erfolgen. Eine kontinuierliche Induktion des Zelltodes kann beispielsweise mittels einer permanent geöffneten Leitung von der Einrichtung 1 zur Biomasseerzeugung zur Einrichtung 21 zur thermischen Induktion der Spezies 16 durchgeführt werden. Auf diese Weise kann das Medium 3 mit der Spezies 16 kontinuierlich durch die Einrichtung 21 zur thermischen Induktion des Zelltodes strömen.

Generell kann vorgesehen sein, dass die Strömungsgeschwindigkeit oder der Volumenstrom des Mediums 3 mit der Spezies 16 gesteuert oder geregelt wird. Weiterhin ist es möglich, dass die Strömung des Mediums 3 durch die Einrichtung 21 temporär unterbrochen wird, insbesondere um eine diskontinuierliche bzw. batchweise Induktion des Zelltodes zu ermöglichen. Auch ist ein Wechsel zwischen diskontinuierlicher und kontinuierlicher Induktion möglich.

Die der Einrichtung 21 nachgeordnete, insbesondere daran angeschlossene Sedimentationseinrichtung 22 dient der gravimetrischen Separation der Spezies 16 vom Medium 3. Die Sedimentationseinrichtung 22 kann mit der Einrichtung 21 eine Baueinheit bilden oder in diese integriert sein oder umgekehrt.

Unter gravimetrischer Separation der Spezies 16 wird ein durch die Gewichtskraft verursachtes Herabsinken (Sedimentieren) der toten bzw, in ihrer Vitalität reduzierten Spezies 16 in Flüssigkeiten, hier im Medium 3, vorzugsweise Wasser, bezeichnet. Im lebenden Zustand ist die Spezies 16, insbesondere Algen, befähigt, sich beispielsweise mit Hilfe feinster Cilien bzw. Haaren an der Zelloberfläche an der Oberfläche des Mediums 3 zu halten, um Photosynthese betreiben zu können, Spezies 16, die keine Cilien bzw. Haare aufweisen, können ihre Position an der Oberfläche des Mediums 3 aufgrund ihrer metabolischen Aktivität, insbesondere aufgrund der Aufnahme und Produktion von gasförmigen Substanzen, halten. Sämtliche Vitalfunktionen, insbesondere die Benutzung der Haare bzw. Cilien zum Schwimmen bzw. die metabolische Aktivität, werden durch eine Induktion des Zelltodes, insbesondere die thermische Induktion des Zelltodes, unterbunden oder zumindest vermindert, so dass die Spezies 16 in dem Medium 3 sedimentiert.

Der Begriff "tote Spezies" umfasst vorzugsweise neben tatsächlich abgestorbenen Spezies auch solche Spezies, deren Vitalfunktionen zumindest zeitweise derart beeinträchtigt sind, dass ihr Sedimentationsverhalten dem von tatsächlich abgestorbenen Zellen zumindest im Wesentlichen entspricht.

Nach der Induktion des Zelltodes erfolgt vorzugsweise die gravimetrische Sedimentation. Diese kann in der Einrichtung 21 selbst und/oder in der nachgeordneten Sedimentationseinrichtung 21 erfolgen. Alternativ kann die Einrichtung 21 auch in die Sedimentationseinrichtung 22 integriert sein.

Nach der thermischen Induktion des Zelltodes der Spezies wird das Medium 3 mit der Spezies 16 zur gravimetrischen Separation von Medium 3 und Spezies 16 in die Sedimentationseinrichtung 22 überführt. Dies kann kontinuierlich oder diskontinuierlich erfolgen, so dass in der Folge die gravimetrische Separation ebenfalls kontinuierlich oder diskontinuierlich erfolgen kann. Vorzugsweise weist die Sedimentationseinrichtung 21 einem Behälter zur Aufnahme des in der Einrichtung 21 behandelten Mediums 3 auf.

Um feststellen zu können, wie weit die Separation der Spezies 16 vom wässrigen Medium 3 fortgeschritten ist, ist es erfindungsgemäß besonders bevorzugt, wenn die Sedimentationseinrichtung 22 bzw. der Behälter transparent ausgebildet oder mit einem Sichtfenster ausgestattet ist. Alternativ oder zusätzlich kann die Sedimentationseinrichtung 22 mit einem oder mehreren Sensoren zur Messung der optischen Dichte des Medium 3 mit der nunmehr toten Spezies, insbesondere im Bodenbereich und/oder im oberen Bereich, ausgestattet sein. Je weiter die optische Dichte des Mediums 3 im oberen Bereich der Sedimentationseinrichtung 22 sinkt und/oder je stärker die optische Dichte im unteren Bereich der Sedimentationseinrichtung 22 ansteigt, desto weiter ist die Separation bzw. Sedimentation fortgeschritten.

Vorzugsweise bietet eine Rückleitung 24 zur Einrichtung 1 und/oder zu einem zusätzlichen Zwischenspeicher die Möglichkeit einer Rezyklisierung des separierten Wassers bzw. Mediums 3. Dabei ist es auch möglich, dass die Rückleitung 24 bzw. Rückführung oder der Zwischenspeicher ein Filter aufweist, um unerwünschte Substanzen vor der Rezyklisierung aus dem Medium 3 zu entfernen. Was die Ausgestaltung bzw. die Feinheit des Filters betrifft, so kann diese variabel sein und in Abhängigkeit von den abzutrennenden Substanzen ausgewählt werden. Beispiele für solche Substanzen können im Rahmen der gravimetrischen Separation nicht abgetrennte Spezies 16 sein. Es kann sich aber auch um Stoffwechselprodukte oder sonstige während der vorangegangenen Verfahrensschritte entstandene Stoffwechselprodukte der Spezies handeln. Besonders bevorzugt weist die Rückleitung 24 bzw. Rückführung oder der Zwischenspeicher ein Sterilfilter auf, um etwaige Kontaminationen, insbesondere durch Mikroorganismen, wie Bakterien oder Hefen, vor einem erneuten Einsatz des Mediums 3 zu entfernen.

Vorzugsweise werden durch die gravimetrische Sedimentation der Spezies 16 mindestens 70 Gew.-%, vorzugsweise mindestens 80 Gew.-%, insbesondere mindestens 85 Gew.-% oder sogar etwa 95 Gew,-%, des Mediums 3 von der Spezies 16 separiert.

Die Entnahme der Biomasse bzw. der sedimentrierten / aufkonzentrierten Spezies 16 aus der Sedimentationseinrichtung 22 kann auf verschiedene Art und Weise erfolgen. So kann die Sedimentationseinrichtung 22 beispielsweise im Bereich des Bodens einen Auslass, insbesondere mit einem zugeordneten Ventil aufweisen, besitzen, über welchen die sedimentierte Spezies 16 abfließen kann. Dabei ist es besonders vorteilhaft, wenn das Ventil in Abhängigkeit von der optischen Dichte im Bodenbereich der Sedimentationseinrichtung 2 vorzugsweise automatisch gesteuert wird. Auf diese Weise kann die Separationseffizienz erhöht werden, indem bei der Entnahme der Biomasse bzw. Spezies 16 die gleichzeitige Entnahme von Medium 3 minimiert wird. Insbesondere ist es auf diese Weise auch möglich, Fraktionen mit unterschiedlicher Zelldichte zielgerichtet zu entnehmen.

Weiter ist es auch möglich, dass die Sedimentationseinrichtung 22 einen Auslass aufweist, welcher so positioniert ist, dass das überschüssige Medium 3 nach der Sedimentation abfließen bzw. abgeleitet werden kann, die Biomasse bzw. sedimentierte Spezies 16 jedoch - zumindest im Wesentlichen - auf dem Boden und/oder im unteren Bereich der Sedimentationseinrichtung 22 verbleibt.

Eine weitere Möglichkeit besteht darin, das Medium 3 über eine Öffnung im oberen Bereich der Sedimentationseinrichtung 22 abzusaugen, so dass die Biomasse im unteren Teil der Sedimentationseinrichtung 22 zurückbleibt, Generell kann es vorgesehen sein, dass der Prozess der Biomasseentnahme bzw. Entnahme des Mediums 3 unter Messung der optischen Dichte kontrolliert, insbesondere gesteuert oder geregelt, abläuft.

Mit der gravimetrischen Separation ist eine vollständige Entfernung des Mediums 3 von der Spezies 16 nicht möglich. Aus diesem Grund kann die erfindungsgemäße Vorrichtung 20 zur Biomasseerzeugung bzw. die vorgeschlagene gravimetrische Separation auch mit einem weiteren Separationsschritt oder Trocknungsschritt bzw, mit einer Trocknungseinrichtung 23 zur weiteren Abtrennung des Mediums 3 bzw. zur zumindest im Wesentlichen vollständigen Trocknung der Spezies 16 kombiniert werden.

Mittels der Trocknungseinrichtung 23 kann das nach der gravimetrischen Separation verbliebene Medium 3 weiter und insbesondere zumindest im Wesentlichen vollständig entfernt bzw. die Spezies 16 (weiter) getrocknet werden.

Besonders bevorzugt weist die Trocknungseinrichtung 23 Folien, Filter mit Poren oder dergleichen auf, auf welche die Biomasse gepresst wird. Die Flüssigkeit bzw. das Wasser wird der Biomasse insbesondere durch das Wirken von Kapillarkräften über die Poren entzogen.

Auch ist es möglich, das verbliebene Medium 3 mittels Lyophilisation zu entfernen. Dies ist insbesondere im Hinblick auf eine Nutzung der Biomasse bzw. Spezies 16 für besonders hochwertige Produkte, wie Nahrungsmittel, welche die Inhaltsstoffe der Algen bzw. Spezies 16 in nahezu unveränderter Form aufweisen sollen, relevant, da die Lyophilisation ein besonders schonendes Verfahren darstellt. Weiterhin ist es möglich, dass das Medium 3 durch Verdunstung und/oder Verdampfung, beispielsweise in einem Ofen, entfernt wird.

Eine weitere Möglichkeit hinsichtlich einer zumindest im Wesentlichen vollständigen Entfernung des Mediums 3 besteht in der Ultrazentrifugation.

Auch die Trocknungseinrichtung 23 kann zur Rezyklisierung über eine Rückleitung 25 direkt mit der Einrichtung 1 und/oder mit der Rückleitung 24 und/oder mit einem zusätzlichen Zwischenspeicher verbunden sein. Auch kann es bei der Rückleitung 25 vorgesehen sein, dass sie mindestens ein Filter aufweist, wie bereits im Zusammenhang mit der Rückleitung 24 beschrieben.

Sämtliche Einrichtungen der Vorrichtung 20 können vorzugsweise direkt und/oder indirekt miteinander verbunden sein. Auch können sie eine Baugruppe bilden und/oder als unabhängige Einrichtungen realisiert werden.

Fig. 3 zeigt in einer detaillierteren Darstellung einen bevorzugten Aufbau der Vorrichtung 20 und insbesondere der Einrichtung 21 zur thermischen Induktion des Zelltodes,

In der Einrichtung 21 zur thermischen Induktion des Zelltodes erfolgt vorzugsweise eine Erwärmung des Mediums 3 mit der Spezies 16. Insbesondere erfolgt die Erwärmung unter Nutzung solarthermischer Energie bzw. durch Sonnenlicht 2. Dabei kann jede geeignete Einrichtung zur Sammlung der im Sonnenenergie verwendet werden, beispielsweise auch - insbesondere handelsübliche - thermische Solarkollektoren.

Vorzugsweise weist die Einrichtung 21 beim Darstellungsbeispiel eine Parabolrinne 26 und/oder ein Kollektorrohr 27 zur Induktion des Zelltodes bzw. Erwärmung des Mediums 2 bzw. der Spezies 16 auf. Insbesondere wird die thermische Strahlung bzw. das Licht 2 mit Hilfe der Parabolrinne 26 gesammelt und in den Brennpunkt der Parabolrinne 26 bzw. auf das Kollektorrohr 27 gelenkt. Vorzugsweise ist die Einrichtung 1 zur Biomasseerzeugung direkt an das Kollektorrohr 27 angeschlossen. Eine indirekte Verbindung von Einrichtung 1 zur Biomasseerzeugung und Kollektorrohr 27 ist aber auch möglich.

So ist die Erwärmung des Mediums 3 und damit der Spezies 16 unter Nutzung solarthermischer Energie möglich. Bei der bevorzugten bzw. dargestellten Ausführungsform handelt es sich um einen energetisch äußerst effizienten Prozess, wie durch die nachfolgende Beispielrechnung veranschaulicht wird:
Für sonnenreiche Gebiete kann unter Hinzunahme der IR-Strahlung von einer solaren Leistung von etwa 1 kW/m² ausgegangen werden. Im Allgemeinen liegt die für die Kultivierung von Algen erforderliche Temperatur bei circa 30 °C.

Die Energiebilanz des erfindungsgemäßen Verfahrens bzw. der erfindungsgemäßen Vorrichtung 20 oder Einrichtung 21 kann wie folgt ermittelt werden:
Mit Hilfe der Gleichung Ws = η x Ps x As x Δt kann ermittelt werden, wie groß die Menge an thermischer Energie ist, die empfangen werden kann. Dabei bezeichnet *η* die Effizienz bzw, den Wirkungsgrad der Parabolrinne, Ps die solare Leistung, As die Fläche der Parabolrinne und Δt das Zeitintervall. Bei einer solaren Leistung von 1 kW/m², einem Wirkungsgrad von *η* = 0,7, einer Fläche der Parabolrinne von 2 m² sowie einem Zeitintervall von 3.600 s beträgt die Menge an aufgenommener Energie etwa 5,04 x 10⁶J bzw. 5,04 x 10⁶ Ws. Diese Energiemenge ist ausreichend, um etwa 48,1 1 des Mediums 3 innerhalb von 1 h um etwa 25 K zu erwärmen. Mit Hilfe der erfindungsgemäßen Vorrichtung 20 bzw. des erfindungsgemäßen Verfahrens können somit etwa 48 1/h bei einer Parabolrinnenfläche von etwa 2 m² - beispielsweise von 30 °C auf etwa 55 °C - erwärmt werden, was die hohe Effizienz des Verfahrens veranschaulicht.

Insbesondere weist die Vorrichtung 20 eine Steuereinheit oder Steuerung auf, welche den Strom des Mediums 3 mit der Spezies 16 durch das Kollektorrohr 27 bzw. die Einrichtung 21 reguliert, insbesondere steuert oder regelt. Dies kann beispielsweise in Abhängigkeit von der Temperatur des Mediums 3 in der Einrichtung 1, 21 und/oder 22 und/oder von der optischen Dichte des Mediums 3 beispielsweise in der Einrichtung 1 erfolgen. Weiter ist es möglich, dass die Vorrichtung 20 zwischen der Einrichtung 1 zur Biomasseerzeugung und dem Kollektorrohr 27 bzw. der Einrichtung 21 einen Sensor zur durchflusszytometrischen Bestimmung der Zellzahl bzw. -dichte aufweist, beispielsweise um den Strom des Mediums 3 durch die Einrichtung 21 zu steuern oder zu regeln.

Die Aufenthaltsdauer des Mediums 3 mit der Spezies 16 bzw. des (mittleren) Volumenstroms im Kollektorrohr 26 bzw. in der Einrichtung 21 ist vorzugsweise derart gewählt oder gesteuert oder geregelt, dass die Erwärmung mit einer Geschwindigkeit von mindestens 1 °C/s, vorzugsweise jedoch mit mindestens 10 °C/s, erfolgt und/oder eine bestimmte Mindesttemperatur im Medium 3 von beispielsweise 50 °C, erreicht wird. Vorzugsweise wird das Medium 3 mit der Spezies 16 bzw. die Spezies 16 mindestens 10 min., vorzugsweise mindestens 30 min., insbesondere für etwa 60 min oder länger erwärmt.

Auch kann das Kollektorrohr 27 entweder direkt oder indirekt mit der Sedimentationseinrichtung 22 in Verbindung stehen oder über eine weitere Leitung verbunden sein. Insbesondere kann es vorgesehen sein, dass der Zustrom des Mediums 3 mit der erwärmten Spezies 16 in die Sedimentationseinrichtung 22 gesteuert und/oder geregelt wird.

In der Sedimentationseinrichtung 22 sinken die abgestorbenen Zellen bzw. Algen zu Boden, da der Zelltod zum Verlust der Motilität führt, während sich das wässrige Medium 3 über den Zellen bzw. Algen absetzt. Durch die Phasentrennung zwischen Biomasse und wässrigem Medium 3 kann die wässrige Phase leicht abgetrennt werden. Erfindungsgemäß kann es dabei vorgesehen sein, dass die Abtrennung entweder durch Absaugen oder Auslassen des Mediums 3 erfolgt oder die Sedimentationseinrichtung 22 im unteren Bereich einen Ablauf zur Entnahme der Biomasse aufweist, wie bereits im Zusammenhang mit Fig. 2 beschrieben.

### Bezugszeichenliste:

- 1: Einrichtung zur Biomasseerzeugung
- 2: (Sonnen)Licht
- 3: Medium
- 4: (lichtleitende) Fasern
- 5: Bündel
- 6: Bündel
- 7: Halterung
- 8: Tank
- 10: Kollektorspiegel
- 11: Versorgungseinrichtung
- 12: Sieb
- 13: Gasblasen
- 14: Zulauf
- 15: Ablauf
- 16: Spezies
- 20: Vorrichtung zur Biomasseerzeugung
- 21: Einrichtung zur thermischen Induktion des Zelltodes
- 22: Sedimentationseinrichtung
- 23: Trocknungseinrichtung
- 24: Rückleitung
- 25: Rückleitung
- 26: Parabolrinne
- 27: Kollektorrohr

## Patentansprüche

1. Verfahren zur Biomasseerzeugung, mit einem vorzugsweise wässrigen Medium (3) mit einer lebenden Spezies (16), insbesondere Algen,
**dadurch gekennzeichnet,**
**dass** der Zelltod der Spezies (16) thermisch induziert wird, um die Spezies (16) vom Medium (3) gravimetrisch zu separieren.

2. Verfahren nach Anspruch 1, **dadurch gekennzeichnet, dass** die Spezies (16) vor Induktion des Zelltodes einer Dauerkultur oder einer Batch-Kultur der Spezies (16) entnommen wird, und/oder dass die thermische Induktion des Zelltodes der Spezies (16) kontinuierlich oder diskontinuierlich durchgeführt wird.

3. Verfahren nach einem der voranstehenden Ansprüche, **dadurch gekennzeichnet, dass** die thermische Induktion des Zelltodes durch Erwärmen der Spezies (16) durchgeführt wird, insbesondere wobei die Spezies (16) mittels Sonneneinstrahlung und/oder Mikrowellenstrahlung erwärmt wird.

4. Verfahren nach Anspruch 3, **dadurch gekennzeichnet, dass** die Spezies (16) auf mindestens 40 °C, insbesondere mindestens 50 °C, erwärmt wird, und/oder dass die Spezies (16) für mindestens 10 min, vorzugsweise mindestens 30 min, insbesondere mindestens 60 min, und/oder dass die Spezies (16) mit einer Geschwindigkeit von mindestens 1 °C/s, insbesondere mindestens 10 °C/s, erwärmt wird.

5. Verfahren nach Anspruch 3 oder 4, **dadurch gekennzeichnet, dass** die Spezies (16) in einer Parabolrinne erwärmt wird, und/oder dass das die Spezies (16) enthaltende Medium (3) nach der Erwärmung in eine Sedimentationseinrichtung (22) geleitet wird.

6. Verfahren nach einem der voranstehenden Ansprüche, **dadurch gekennzeichnet, dass** die Spezies (16) nach der thermischen Induktion des Zelltodes und/oder nach dem Absterben gravimetrisch vom Medium (3) separiert wird, insbesondere wobei die gravimetrische Separation separierte Spezies (16) getrocknet wird, und/oder dass das wässrige Medium (3) nach der Separation der Spezies (16) rezyklisiert wird, insbesondere wobei das nach gravimetrischer Trennung verbliebene Medium (3) zumindest im Wesentlichen von der separierten Spezies (16) getrennt wird.

7. Verfahren nach einem der voranstehenden Ansprüche, **dadurch gekennzeichnet, dass** die Spezies (16) diskontinuierlich oder kontinuierlich vom Medium (3) separiert wird, und/oder dass mindestens 70 Gew.-%, vorzugsweise mindestens 80 Gew.-%, insbesondere mindestens 85 Gew.-%, des Mediums (3) von der Spezies (16) getrennt werden.

8. Vorrichtung zur Biomasseerzeugung (20), mit einem vorzugsweise wässrigen Medium (3) mit einer lebenden Spezies (16), insbesondere Algen,
**dadurch gekennzeichnet,**
**dass** die Vorrichtung (20) eine Einrichtung (21) zur thermischen Induktion des Zelltodes bei der Spezies (16) aufweist, um die Spezies (16) gravimetrisch zu separieren.

9. Vorrichtung nach Anspruch 8, **dadurch gekennzeichnet, dass** die Einrichtung (21) zur Erwärmung der Spezies (16) ausgebildet ist.

10. Vorrichtung nach Anspruch 9, **dadurch gekennzeichnet, dass** die Einrichtung (21) zur Erwärmung der Spezies (16) mittels Sonneneinstrahlung und/oder Mikrowellenstrahlung ausgebildet ist.

11. Vorrichtung nach Anspruch 10, **dadurch gekennzeichnet, dass** die Einrichtung (21) zur Erwärmung der Spezies (16) eine Parabolrinne (26) und/oder ein durch Einstrahlung aufwärmbares Kollektorrohr (27) aufweist.

12. Vorrichtung nach einem der Ansprüche 8 bis 11, **dadurch gekennzeichnet, dass** die Vorrichtung (20) eine Sedimentationseinrichtung (22) zur gravimetrischen Separation der Spezies (16) vom Medium (3) aufweist.

13. Vorrichtung nach Anspruch 12, **dadurch gekennzeichnet, dass** das Medium (3) mit der Spezies (16) in die Sedimentationseinrichtung (22) leitbar ist.

14. Vorrichtung nach einem der Ansprüche 8 bis 13, **dadurch gekennzeichnet, dass** die Vorrichtung (20) zur Rezyklierung des wässrigen Mediums (3) nach der Separation der Spezies (16) vom Medium (3) ausgebildet ist.

15. Vorrichtung nach einem der Ansprüche 8 bis 14, **dadurch gekennzeichnet, dass** die Vorrichtung (20) eine Trocknungseinrichtung (23) aufweist.
